# EUROPÄISCHE PATENTSCHRIFT

(11) **EP 0 806 955 B1**
(45) Veröffentlichungstag und Bekanntmachung des Hinweises auf die Patenterteilung: **17.03.2004**
(21) Anmeldenummer: 95943218.8
(22) Anmeldetag: 28.12.1995
(51) Int. Cl.: A61K 31/70, A61K 31/495

(54) **ENROFLOXACIN-INJEKTIONS- ODER INFUSIONSLÖSUNGEN**
ENROFLOXACINE INJECTION OR INFUSION SOLUTIONS
SOLUTIONS D'INJECTION OU DE PERFUSION D'ENROFLOXACINE

(30) Priorität: 13.01.1995 DE 19500784
(43) Veröffentlichungstag der Anmeldung: 19.11.1997
(73) Patentinhaber: Bayer HealthCare AG, 51368 Leverkusen (DE)
(72) Erfinder: BONSE, Gerhard, D-51061 Köln (DE); HAMM, Martin, D-40593 Düsseldorf (DE); MÜLLER, Hanns-Peter, D-51519 Odenthal (DE); NAIK, Arundev, Haribhai, D-40789 Monheim (DE); SCHEER, Martin, D-42113 Wuppertal (DE); STEGEMANN, Michael, D-40593 Düsseldorf (DE); VETTER, Oliver, D-51399 Burscheid (DE)
(86) Internationale Anmeldenummer: PCT/EP1995/005147
(87) Internationale Veröffentlichungsnummer: WO 1996/021453

(56) Entgegenhaltungen:
- EP-A- 0 219 784
- JOURNAL OF CHROMATOGRAPHY B BIOMEDICAL APPLICATIONS, 658 (2). 1994. 341-348., XP000572835 TYCZKOWSKA K L ET AL: "Simultaneous determination of enrofloxacin and its primary metabolite ciprofloxacin in bovine milk and plasma by ion-pairing liquid chromatography"

## Beschreibung

Die vorliegende Erfindung betrifft neue Injektions- oder Infusionslösungen von Enrofloxacin:

Parenteral verabreichbare Lösungen von Chinoloncarbonsäuren sind bereits bekannt aus EP-OS 67 666. Sie basieren auf Salzen der entsprechenden Chinoloncarbonsäuren mit verschiedenen Hydroxycarbonsäuren.

Injektions- oder Infusionslösungen von Chinoloncarbonsäuren u.a. von Ciprofloxacin auf Basis der milchsauren Salze mit einem Überschuß an Säure sind bekannt aus EP-OS 138 018.

Wäßrige Infusionslösungen von ca. 0,01 bis 0,5 % Ciprofloxacin denen zur Stabilisierung Säuren zugesetzt sind, sind bekannt aus EP-OS 219 784. Als bevorzugte Säure ist darin vor allem Milchsäure genannt.

Injektionslösungen auf Basis von feinverteilten Suspensionen von Chinoloncarbonsäuren sind bekannt aus DE-OS 39 02 079.

Die vorliegende Erfindung betrifft neue Injektions- und Infusionslösungen der Zusammensetzung
a) Enrofloxacin 0,1 bis 20 Gew.-%, bezogen auf das Gesamtgewicht der Lösung in Form seiner Salze mit Gluconsäure, Glucuronsäure, Glutaminsüre, Weinsäure oder deren Mischungen;
b) Gluconsäure, Glucuronsäure, jeweils gegebenfalls in Lactonform Glutaminsäure oder Weinsäure odere deren Mischungen in 0,1 bis 5 molarem Überschuß, bezogen auf Enrofloxacin;
c) gegebenenfalls 0,1 bis 30 Gew.-% Formulierungshilfsmittel, bezogen auf das Gesamtgewicht der Lösung;
d) Wasser bis 100 Gew.-%.

Die erfindungsgemäßen Lösungen haben im Vergleich zu den bis jetzt bekannten Injektionslösungen von Enrofloxacin den Vorteil einer schnell einsetzenden starken Wirkung. Diese zeigt sich in kurzfristig nach Applikation erreichten hohen Blutspiegelwerten des Wirkstoffs. Der Wirkstoff wird aber auch rasch wieder ausgestoßen, was vor allem bei der Behandlung von Tieren, die der Erzeugung von Nahrungsmitteln dienen, von großem Vorteil ist (kurze Wartezeiten).

Die erfindungsgemäßen Injektions- und Infusionslösungen enthalten den Wirkstoff Enrofloxacin in Konzentrationen von bevorzugt 1 bis 10 %, besonders bevorzugt von 2,5 bis 10 % (Gewichtsprozent bezogen auf das Gesamtgewicht der Lösung).

Gluconsäure, Glucuronsäure, können offenkettig vorliegen oder in Form ihrer Lactone. Werden die Säuren als Lactone eingesetzt, wird der Lactonring mindestens teilweise hydrolysiert bevor es zu einer Salzbildung und Komplexbildung mit Enrofloxacin kommt. Gluconsäure, Glucuronsäure, jeweils gegebenenfalls in Lactonform, Glutaminsäure oder Weinsäure oder deren Mischungen liegen in 0,1 bis 5 molarem Überschuß, bezogen auf die Menge Enrofloxacin in der Lösung vor. Bevorzugt ist ein 0,2 bis 2 molarer Überschuß, besonders bevorzugt ein 0,5 bis 1 molarer Überschuß der Säure.

Die Erfindung betrifft außerdem ein Verfahren zur Herstellung von Injektions- und Infusionslösungen der Zusammensetzung
a) Enrofloxacin 0,1 bis 20 Gew.-%, bezogen auf das Gesamtgewicht der Lösung in Form seiner Salze mit Gluconsäure, Glucuronsäure, Glutaminsäure, Weinsäure oder deren Mischungen
b) Gluconsäure, Glucuronsäure, jeweils gegebenenfalls in Lactonform, Glutaminsäure oder Weinsäure, oder deren Mischungen in 0,1 bis 5 molarem Überschuß, bezogen auf Enrofloxacin;
c) gegebenenfalls 0,1 bis 30 Gew.-% Formulierungshilfsmittel bezogen auf das Gesamtgewicht der Lösung;
d) Wasser bis 100 Gew.-%,
dadurch gekennzeichnet, dass man Enrofloxacin in eine wässrige Lösung der Säure einrührt und die übrigen Formulierhilfsmittel zusetzt und gegebenenfalls auf die gewünschte Konzentration mit Wasser verdünnt.

Die wäßrigen Lösungen zur Injektion können als Trägerflüssigkeit neben Wasser für Injektionszwecke weiterhin beispielsweise Ethanol; Glycerin, Propylenglykol, Polyethylenglykol und Triethylenglykol enthalten. Zur Einstellung des pH-Wertes möglichst im Bereich von pH 3 bis 6,5 können unterschiedliche Substanzen wie Phosphorsäure, Zitronensäure, Tris, Ascorbinsäure, Essigsäure, Bernsteinsäure, Weinsäure, Gluconsäure und Milchsäure und ihre Salze eingesetzt werden. Der pH-Wert der wäßrigen Formulierungen gemäß der Erfindung beträgt 3 bis 5,5, vorzugsweise 3,5 bis 5. Die Osmolalität der wäßrigen Suspensionen beträgt 200 bis 900 m Osmol/kg, bevorzugt 260 bis 390 m Osmol/kg, und kann durch Zusatz von NaCl, Glucose, Fructose, Glycerin, Sorbit, Mannit, Saccharose oder Xylit bzw. Mischungen dieser Substanzen an isotone Verhältnisse angepaßt werden

Des weiteren können Formulierungshilfsmittel wie Verdickungsmittel (z.B. Methylcellulose, Hydroxyethylcellulose, Hydroxypropylcellulose, Natriumcarboxymethylcellulose, Polyvinylpyrrolidon, Gelatine u.a.), Resorptionsmittel, Lichtschutzmittel, Kristallisationsverzögerer, Komplexierungsmittel (z.B. NaEDTA, Phosphate, Nitrate, Acetate, Citrate u.a.), Antioxidantien (Ascorbinsäure, Sulfit-Verbindungen, L-Cystein, Thiodipropionsäure, Thiomilchsäure, Monothioglycerin, Propylgallat u.a.) sowie Konservierungsmittel (PHB-Ester, Phenol und Derivate, Chlorbutanol, Benzylalkohol, Ethanol, Butanol, 1,3-Butandiol, Chlorhexidin-Salze, Benzoesäure und Salze, Sorbinsäure u.a.) eingesetzt werden. Gegebenenfalls können Lokalanästhetika wie etwa Procain-HCl, Lidocain-HCl und andere den erfindungsgemäßen Lösungen zugesetzt werden.

Zur Herstellung der erfindungsgemäßen Injektions- oder Infusionslösungen kann man vom Enrofloxacinsaiz mit der entsprechenden Säure oder einem Hydrat desselben ausgehen.

Man kann aber auch die Salze direkt in der Lösung herstellen, und zwar durch Zugabe der zur Salzbildung erforderlichen Mengen Säure. Zur Beschleunigung des Auflösens kann bei Temperaturen zwischen 30 bis 60°C gearbeitet werden. Die Herstellung der Lösungen kann unter Stickstoff-Begasung erfolgen.

In dieser Weise kann man sowohl gebrauchsfertige Lösungen der aktiven Substanz, abgefüllt in geeignete Behälter, z.B. in. Ampullen, Injektions- oder Infusionsflaschen, als auch zur Herstellung solcher Lösungen geeignete Vorprodukte z.B. Konzentrate oder Lyophilisate herstellen.

Die Behältnisse, in welche die Zubereitungen abgefüllt werden, können sowohl aus Glas als auch aus Kunststoff bestehen. Dabei können die Behältermaterialien Substanzen enthalten, die dem Inhalt einen besonderen Schutz verleihen, wie z.B. einen Lichtschutz oder einen Sauerstoffschutz.

Die Herstellung der Lösungen in den folgenden Beispielen kann in Ansatzkesseln mit oder ohne Temperiermantel erfolgen. Bei Verwendung eines nicht heizbaren Kessels kann erforderlichenfalls vorgewärmtes Wasser eingesetzt werden.

Die Einzelkomponenten werden im allgemeinen nach Vorlegen der Hauptmenge des Lösungsmittels in diesem gelöst. Das Lösungsmittel kann jedoch auch zu den Feststoffen zugegeben werden.

Bei Verwendung von Säuren, die in Form ihrer Lactone eingesetzt werden, werden diese nach dem Auflösen im allgemeinen durch Erwärmen oder Stehenlassen zur freien Säure hydrolysiert, bevor der Wirkstoff zugegeben wird. Dabei wird eine Temperatur von 40-70°C verwendet, vorzugsweise 50-60°C.

Die weiteren Bestandteile werden im Anschluß daran in der Zubereitung vor oder nach dem Abkühlen unter Rühren gelöst bzw. in diese eingearbeitet. Nach Ergänzen mit der Restmenge des Lösungsmittels kann die Formulierung durch geeignete bakterienzurückhaltende Filter sterilfiltriert und/oder hitzesterilisiert werden.

### Beispiele

| **Beispiel 1** | | |
|---|---|---|
| | g/100 ml | |
| Enrofloxacin 100 % | 10,00 | |
| Gluconolacton | 8,00 | |
| Benzylalkohol doppelt dest. | 1,40 | |
| Natriumsulfit | 0,10 | |
| Wasser für Injektionszwecke | 86,70 | |
| | 100 ml = 106,20 | pH 3,90 |

| **Beispiel 2** | | |
|---|---|---|
| | g/100 ml | |
| Enrofloxacin 100 % | 5,00 | |
| Gluconolacton | 3,00 | |
| Benzylakohol | 1,00 | |
| Wasser für Injektionsszwecke | 93,60 | |
| | 100 ml = 102,60 | pH 4,40 |

| **Beispiel 3** | | |
|---|---|---|
| | g/100 ml | |
| Enrofloxacin 100 % | 5,00 | |
| Glucuronsäure | 3,25 | |
| Benzylalkohol | 1,00 | |
| Wasser für Injektionszwecke | 93,45 | |
| | 100 ml = 102,70 | pH 3,85 |

| **Beispiel 4** | | |
|---|---|---|
| | g/100 ml | |
| Enrofloxacin 100 % | 5,00 | |
| L-Glutaminsäure reinst | 2,50 | |
| Benzylalkohol | 1,00 | |
| Wasser für Injektionszwecke | 93,80 | |
| | 100 ml = 102,30 | pH 5,15 |

| **Beispiels 5** | | |
|---|---|---|
| | g/100 ml | |
| Enrofloxacin 100 % | 5,00 | |
| Weinsäure | 1,25 | |
| Benzylalkohol | 1,00 | |
| Wasser für Injektionszwecke | 94,55 | |
| | 100 ml = 101,80 | pH 4,40 |

| **Beispiel 6** | | |
|---|---|---|
| | g/100 ml | |
| Enrofloxacin 100 % | 10,000 | |
| Gluconolacton | 4,000 | |
| Essigsäure 100 % | 1,175 | |
| Benzylalkohol | 1,400 | |
| Natriumsulfit | 0,100 | |
| Wasser für Injektionszwecke | 8,025 | |
| | 100 ml = 104,700 | pH 4,42 |

| **Beispiel 7** | | |
|---|---|---|
| | g/100 ml | |
| Enrofloxacin 100 % | 10,000 | |
| Gluconolacton | 4,000 | |
| Glutaminsäure | 2,670 | |
| Benzylalkohol | 1,400 | |
| Natriumsulfit | 0,100 | |
| Wasser für Injektionszwecke | 87,330 | |
| | 100 ml = 105,500 | pH 4,28 |

## Patentansprüche

1. Injektions- und Infusionslösungen der Zusammensetzung
a) Enrofloxacin 0,1 bis 20 Gew.-%, bezogen auf das Gesamtgewicht der Lösung in Form seiner Salze mit Gluconsäure, Glucuronsäure, Glutaminsäure, Weinsäure oder deren Mischungen;
b) Gluconsäure, Glucuronsäure, jeweils gegebenenfalls in Lactonform, Glutaminsäure oder Weinsäure oder deren Mischungen in 0,1 bis 5 molarem Überschuß, bezogen auf Enrofloxacin;
c) gegebenenfalls 0,1 bis 30 Gew.-% Formulierungshilfsmittel bezogen auf das Gesamtgewicht der Lösung;
d) Wasser bis 100 Gew.-%.

2. Verfahren zur Herstellung von Injektions- und Infusionslösungen der Zusammensetzung
a) Enrofloxacin 0,1 bis 20 Gew.-%, bezogen auf das Gesamtgewicht der Lösung in Form seiner Salze mit Gluconsäure, Glucuronsäure, Glutaminsäure, Weinsäure oder deren Mischungen
b) Gluconsäure, Glucuronsäure, jeweils gegebenenfalls in Lactonform, Glutaminsäure oder Weinsäure, oder deren Mischungen in 0,1 bis 5 molarem Überschuß, bezogen auf Enrofloxacin;
c) gegebenenfalls 0,1 bis 30 Gew.-% Formulierungshilfsmittel bezogen auf das Gesamtgewicht der Lösung;
d) Wasser bis 100 Gew.-%,
**dadurch gekennzeichnet, dass** man Enrofloxacin in eine wässrige Lösung der Säure einrührt und die übrigen Formulierhilfsmittel zusetzt und gegebenenfalls auf die gewünschte Konzentration mit Wasser verdünnt.

## Revendications

1. Solutions administrables par injection et perfusion, de composition suivante :
a) Enrofloxacine 0,1 à 20% en poids, par rapport au mélange total de la solution, sous forme de ses sels d'acide gluconique, d'acide glucuronique, d'acide glutamique, d'acide tartrique ou de leurs mélanges ;
b) acide gluconique, acide glucuronique, chacun éventuellement sous forme de lactone, acide glutamique ou acide tartrique, ou leurs mélanges en excès molaire de 0,1 à 5, par rapport à l'Enrofloxacine ;
c) éventuellement 0,1 à 30% en poids d'auxiliaires de formulation, par rapport au poids total de la solution ;
d) eau, complément à 100% en poids.

2. Procédé de préparation de solutions administrables par injection et perfusion, de composition suivante :
a) Enrofloxacine 0,1 à 20% en poids, par rapport au mélange total de la solution, sous forme de ses sels d'acide gluconique, d'acide glucuronique, d'acide glutamique, d'acide tartrique ou de leurs mélanges ;
b) acide gluconique, acide glucuronique, chacun éventuellement sous forme de lactone, acide glutamique ou acide tartrique, ou leurs mélanges en excès molaire de 0,1 à 5, par rapport à l'Enrofloxacine ;
c) éventuellement 0,1 à 30% en poids d'auxiliaires de formulation, par rapport au poids total de la solution ;
d) eau, complément à 100% en poids,
**caractérisé en ce qu'**on incorpore en agitant l'Enrofloxacine dans une solution aqueuse de l'acide et on ajoute les autres auxiliaires de formulation, et on dilue éventuellement à la concentration voulue avec de l'eau.

## Claims

1. Injection and infusion solutions of the following composition:
a) 0.1 to 20% by weight of enrofloxacin, based on the total weight of the solution, in the form of its salts with gluconic acid, glucuronic acid, glutamic acid, tartaric acid or mixtures thereof;
b) gluconic acid, glucuronic acid, each optionally in lactone form, glutamic acid or tartaric acid or mixtures thereof in 0.1 to 5 molar excess, based on enrofloxacin;
c) optionally 0.1 to 30% by weight of formulation aids, based on the total weight of the solution;
d) water to 100% by weight.

2. Process for the preparation of injection and infusion solutions of the following composition:
a) 0.1 to 20% by weight of enrofloxacin, based on the total weight of the solution, in the form of its salts with gluconic acid, glucuronic acid, glutamic acid, tartaric acid or mixtures thereof
b) gluconic acid, glucuronic acid, each optionally in lactone form, glutamic acid or tartaric acid or mixtures thereof in 0.1 to 5 molar excess, based on enrofloxacin;
c) optionally 0.1 to 30% by weight of formulation aids, based on the total weight of the solution;
d) water to 100% by weight,
**characterized in that** enrofloxacin is stirred into an aqueous solution of the acid, the remaining formulation aids are added and, if appropriate, the desired concentration is obtained by dilution with water.
